# EUROPÄISCHE PATENTSCHRIFT

(11) **EP 0 518 351 B1**
(45) Veröffentlichungstag und Bekanntmachung des Hinweises auf die Patenterteilung: **10.05.1995**
(21) Anmeldenummer: 92109911.5
(22) Anmeldetag: 12.06.1992
(51) Int. Cl.: C07C 229/62, C07C 227/08

(54) **Verfahren zur Herstellung von 2,5-Di-(phenylamino)-terephthalsäure und ihrer Dialkylester in hoher Reinheit**
Process for the preparation of 2,5-di(phenylamino)terephthalic acids and their dialkylesters in high purity
Procédé pour la préparation d'acides 2,5-di(phénylamino)térephthaliques et leurs esters dialkyliques en haute pureté

(30) Priorität: 14.06.1991 DE 4119601
(43) Veröffentlichungstag der Anmeldung: 16.12.1992
(73) Patentinhaber: HOECHST AKTIENGESELLSCHAFT, 65926 Frankfurt am Main (DE)
(72) Erfinder: Fuchs, Hermann, Dr., W-6240 Königstein (DE); Gilb, Walter, Dr., W-6238 Hofheim/Taunus (DE); Arndt, Otto, Dr., W-6238 Hofheim/Tainus (DE)

(56) Entgegenhaltungen:
- EP-A- 0 363 756

## Beschreibung

Die vorliegende Erfindung betrifft ein technologisch vorteilhaftes Verfahren zur Herstellung von 2,5-Di-(phenylamino)-terephthalsäure und ihrer Dialkylester der allgemeinen Formel (I) 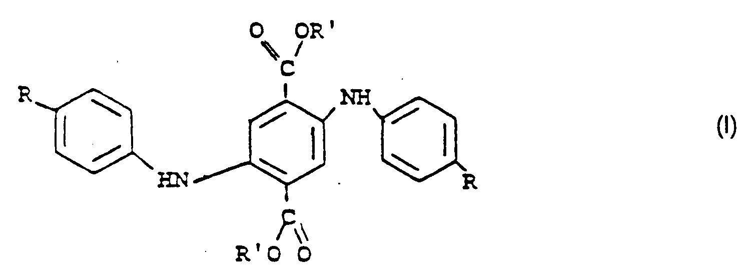
in welcher R ein Wasserstoffatom oder eine Methylgruppe und R' ein Wasserstoffatom oder eine Methyl- oder Ethylgruppe bedeuten.

Es ist bekannt, 2,5-Di-(phenylamino)-terephthalsäure und deren Dialkylester nach einem Mehrstufenverfahren herzustellen, indem man nach Art einer Dieckmann- bzw. doppelten Claisen-Kondensation Bernsteinsäure-dialkylester zum 2,5-Dihydroxy-cyclohexadien-dicarbonsäure-(1,4)-dialkylester cyclisiert (Fortschr. chem. Forschung, Bd. 1 (1950) 685-724), anschließend diesen in den 2,5-Di-phenylamino-dihydro(3,6)-terephthalsäure-dialkylester durch eine Kondensationsreaktion mit einem primären Phenylamin (beispielsweise Anilin oder Toluidin) in Xylol oder Ethylbenzol oder in Gemischen daraus in Gegenwart einer aliphatischen Säure (beispielsweise Essigsäure) überführt, diesen dehydriert (oxidiert) zum 2,5-Di-(phenylamino)-terephthalsäure-dialkylester, anschließend diesen Ester alkalisch verseift (z. B. in alkoholischer Natronlauge) und durch Behandeln des angefallenen 2,5-di-(phenylamino)-terephthalsauren Dinatriumsalzes mit Säure die 2,5-Di-(phenylamino)-terephthalsäure freisetzt.

Bei der Beschreibung der Herstellung der Di-(phenylamino)-terephthalsäure nach dem vorstehend beschriebenen Weg aus Bernsteinsäureester werden in der Literatur (JP 49-108 036; DE-OS 1 915 436 und DE-0S 3 834 747 (EP 0 363 756)), eine Reihe von Verfahrensparametern beschrieben, wie beispielsweise Lösemittel; die Zwischenisolierung einzelner oder aller Synthesestufenprodukte (wie
(1) Succinylo-bernsteinsäure-dialkylester;
(2) 2,5-Di-(phenylamino)-dihydro(3,6)-terephthalsäure-dialkylester;
(3) 2,5-Di-(phenylamino)-terephthalsäure-dialkylester;
(4) 2,5-Di-(phenylamino)-terephthalsäure); die Art der angewandten Katalysatoren, gegebenenfalls mit Zusatzstoffen für die vorstehend genannten Zwischenstufen (1), (2) und (3); zeitliche Abfolge von Oxidation und Verseifung (Verseifung gleichzeitig mit Oxidation oder anschließend); Dehydrierungs-(Oxidations)mittel (wie beispielsweise Nitrobenzol und dessen Derivate, Chinone, Sauerstoff, Jod); Aufarbeitung der eingesetzten Hilfsstoffe (wie beispielsweise Lösemittel, Phenylamin (Anilin, p-Toluidin), Katalysatoren, Zusatzstoffe).

Gegenstand der vorliegenden Erfindung ist nun ein Verfahren zur Herstellung von 2,5-Di-(phenylamino)-terephthalsäure und ihrer Dialkylester der allgemeinen Formel (I), in welcher R ein Wasserstoffatom oder eine Methylgruppe und R' ein Wasserstoffatom oder eine Methyl- oder Ethylgruppe bedeuten, durch (1) Umsetzung von Bernsteinsäure-dialkyl(C₁-C₂)-ester nach Art einer Dieckmann-Kondensation mit Natrium-alkoholat in Xylol zum Dinatrium-Salz des 2,5-Dihydroxy-cyclohexadien-dicarbonsäure-(1,4)-dialkyl(C₁-C₂)esters, (2) Umsetzung des so erhaltenen Kondensationsprodukts nach Zersetzen des Dinatrium-Salzes mit Säure mit einem Phenylamin der allgemeinen Formel (II), 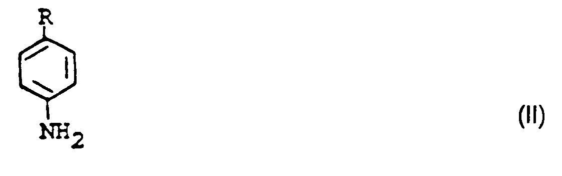
in welcher R die vorstehend genannte Bedeutung hat, in Gegenwart einer organischen Säure in Xylol zum 2,5 Di-(phenylamino)-dihydro(3,6)-terephthalsäuredialkyl(C₁-C₂)-ester, (3) Dehydrierung (Oxidation) des so erhaltenen Cyclohexadien-1,4-derivats mit Sauerstoff zum entsprechenden 2,5-Di-(phenylamino)-terephthalsäure-dialkyl (C₁-C₂)-ester, (4) Verseifung des so erhaltenen Dialkylesters in methanolischer Natronlauge zum entsprechenden 2,5-di-(phenylamino)-terephthalsauren Dinatrium-Salz und (5) Freisetzung der 2,5-Di-(phenylamino)terephthalsäure aus dem genannten Dinatriumsalz mit Säure, das dadurch gekennzeichnet ist, daß man die Oxidation in Stufe (3) so durchführt, daß man das als Lösung oder Suspension vorliegende Reaktionsgemisch der Stufe (2) in einem Rührkessel mit Stickstoff überlagert, dieses Reaktionsgemisch im Kreislauf umpumpt und den Sauerstoff in ein Teilvolumen des umgepumpten Reaktionsgemisches so eindosiert, daß eine effektive Durchmischung erfolgt.

Der Sauerstoff kann dabei allein oder im Gemisch mit inerten Gasen, z. B. in Form von Luft, eindosiert werden. Das Xylol kann allein oder in Form von technischen Xylolgemischen eingesetzt werden.

Für die Umsetzung mit dem Phenylamin der Formel II in Stufe (2) kann beispielsweise Propionsäure oder Hexafluorpropansulfonsäure als Säurekatalysator verwendet werden.

Es ist darüber hinaus von Vorteil, gleichzeitig mit der Eindosierung des Sauerstoffs noch vorhandene Feststoffteilchen der Suspension zu zerkleinern.

Sowohl eine effektive Durchmischung des umgepumpten, sich in dem Teilvolumen befindlichen Reaktionsgemisches mit dem Sauerstoff als auch die Zerkleinerung evtl. vorhandener Kristalle kann vorteilhaft durch Kavitationseffekte, die beispielsweise eine Kreiselhomogenisiermaschine erzeugt, bewerkstelligt werden.

Bei Verwendung einer Kreiselhomogenisiermaschine mit Eindüsung ist der Funktionsraum dieser Pumpe, in den durch eine Zuleitung das Gas eindosiert wird, gleichzeitig das Teilvolumen, in dem die Reaktion stattfindet. Der Funktionsraum besteht aus einem ineinandergreifenden Rotor-Stator-System. Rotoren und Statoren besitzen Durchtrittsöffnungen, beispielsweise in Form von Schlitzen oder zylindrischen Durchlässen. Die Durchtrittsöffnungen füllen sich mit Bearbeitungsgut in Form einer Lösung oder Suspension von 2,5-Diphenylamino-dihydro(3,6)-terephthalsäureester und eingedüstem gasförmigem Sauerstoff. Der mit hoher Geschwindigkeit drehende Rotor preßt das Bearbeitungsgut durch die hohe Zentrifugalbeschleunigung aus den Durchtrittsöffnungen in die Statorräume. Während der Befüllung werden die Statorräume durch den nächst größeren Rotorring geschlossen, so daß das Bearbeitungsgut nicht austreten kann. In den Statorräumen herrscht ein erheblicher Überdruck, der innerhalb von Sekundenbruchteilen durch das Weiterdrehen des Rotors schockartig entspannt wird. Dadurch werden extreme Kavitationseffekte erreicht, wodurch eine äußerst effektive Verteilung des Sauerstoffs ermöglicht wird. Gleichzeitig werden suspendierte Kristalle von 2,5-Diphenylamino-dihydro(3,6)-terephthalsäureester wirkungsvoll zerkleinert, so daß deren spezifische Oberfläche erheblich vergrößert und damit die Reaktionsgeschwindigkeit erhöht wird.

Bei dem erfindungsgemäßen Verfahren kann bei der Oxidation auf den Einsatz eines Katalysators verzichtet werden, was einen besonderen Vorteil darstellt. Es können aber auch Katalysatoren aus V₄A-Stahl und/oder aus einem Übergangsmetall des Periodischen Systems der Elemente und/oder aus einem seltenen Erdmetall mit variablen Oxidationsstufen oder dessen Verbindungen oder Mischungen daraus eingesetzt werden.

Ein Vorteil des erfindungsgemäßen Verfahrens ist die relativ kurze Reaktionszeit verbunden mit günstigen Raum-Zeit-Ausbeuten. Dies beruht unter anderem darauf, daß das Teilvolumen, in dem die Reaktion mit dem Sauerstoff stattfindet, sehr viel kleiner ist als das Volumen des Rührkessels, das zwangsläufig so groß sein muß, daß es das gesamte im Produktionsverbund anfallende Produkt der Stufe (2) aufnehmen kann. Ein solches Teilvolumen kann nämlich aufgrund seiner geringeren Größe ohne größeren Aufwand explosionssicher ausgelegt sein und damit einen sehr viel größeren Sauerstoffgehalt als den für das Gasgemisch über dem Reaktionsgemisch im Rührkessel und im Kreisgas zulässigen Gehalt von unterhalb 8 Vol.-% zulassen. Dadurch ist die Konzentration an gelöstem Sauerstoff in der Lösung oder Suspension erhöht und damit die Reaktionsgeschwindigkeit.

Erfindungsgemäß wird eine optimale Zerkleinerung der Gasblasen und damit eine größere Fläche, an der der Austausch zwischen Gas und Flüssigkeit stattfinden kann, bewirkt, so daß die Reaktionszeit noch um etwa 60 bis 65 % verkürzt wird.

Die erhöhte Reaktionsgeschwindigkeit ist insbesondere deshalb von Vorteil, weil dadurch optimale Taktzeiten für die jeweilige Weiterverarbeitung beim Übergang von Stufe (2) nach (3) und weiter nach Stufe (4) erreicht werden.

Da die Reaktionszeit auch von der Größe des Teilvolumens abhängig ist, läßt sie sich auf diese Weise steuern, so daß optimale Taktzeiten eingehalten werden können.

### Beispiele

1. In einem 600 l Rührkessel wurden 230 kg Xylol und 27 kg Propionsäure vorgelegt und verrührt. Nach Zugabe von 34,2 kg (150 Mol) Succinmethylester wurde auf 60 °C erwärmt und danach wurden 38,7 kg (361 Mol), bei 80 °C geschmolzenes p-Toluidin eingesaugt. Der Gasraum über diesem Reaktionsgemisch wurde 3 mal mit Stickstoff gespült und der Ansatz am Umlauf gekocht, bis das Kondensationswasser vollständig ausgekreist war. Die Temperatur im Kessel wurde auf 100 °C eingestellt und das Reaktionsgemisch im Kreislauf umgepumpt. In den Kreislauf des umgepumpten Reaktionsgemisches wird eine Kreiselhomogenisierungspumpe eingeschaltet. Es wurden ca. 11 m³/h umgepumpt, d. h. der Inhalt des Rührkessels (ca. 370 l) wurde ca. 30 mal pro Stunde umgepumpt. In die Kreiselhomogenisierungspumpe wurde der Sauerstoff so eindosiert, daß der Sauerstoffgehalt im Kreisgas unter 8 Vol.-% blieb. Gleichzeitig wurde das bei der Oxidation entstehende Wasser azeotrop abdestilliert. Nach 4,5 bis 5 Stunden war die Oxidation beendet. Es wurden 3,3 bis 3,6 kg Sauerstoff (Theorie 2,4 kg) verbraucht und 3,7 bis 4,1 kg Wasser (Theorie 2,7 kg) ausgeschleust.
   Die Ausbeute an 2,5-Di-p-toluidino-terephthalsäure-dimethylester betrug 98 bis 99 % der Theorie (HPLC-analytische Bestimmung in der Reaktionslösung als Flächenprozente).
2. Das Beispiel 1 wurde wiederholt, jedoch mit 33,6 kg (361 Mol) Anilin anstelle des p-Toluidin. Nach ca. 5 Stunden war die Oxidation beendet. Es wurden ca. 3,3 kg Sauerstoff verbraucht und ca. 3,5 kg Wasser ausgeschleust.
   Die Ausbeute an 2,5-Diphenylamino-terephthalsäure-dimethylester betrug über 98 % der Theorie (HPLC-analytische Bestimmung wie im Beispiel 1).
3. In einem 600 l Rührkessel wurde eine technische Rohlösung von Succinmethylester in Xylol, so wie sie im Produktionsprozeß anfiel, vorgelegt. Sie enthielt 34,2 kg (150 Mol) Succinmethylester und wurde mit soviel Xylol versetzt, daß eine dem Beispiel 1 analoge Konzentration von 12,9 % Succinmethylester entstand. Nach Zugabe von 22,4 kg Propionsäure wurde auf 60 °C erwärmt und danach wurden 33,9 kg (316 Mol), bei 80 °C aufgeschmolzenes p-Toluidin eingesaugt. Desweiteren wurde wie im Beispiel 1 verfahren. Nach 6 Stunden war die Oxidation beendet. Es wurden 3,6 kg Sauerstoff verbraucht und 4,2 kg Wasser ausgeschleust.
   Die Ausbeute an 2,5-Di-p-toluidino-terephthalsäure-dimethylester betrug über 98 % der Theorie (HPLC-analytische Bestimmung wie im Beispiel 1).
4. In einem 1500 l Rührkessel wurde eine technische Rohlösung von Succinmethylester in Xylol, so wie sie im Produktionsprozeß anfiel, vorgelegt. Sie enthielt 113,3 kg (496,5 Mol) Succinmethylester und wurde mit soviel Xylol versetzt, daß eine dem Beispiel 1 analoge Konzentration von 12,9 % Succinmethylester entstand. Nach Zugabe von 77,2 kg Propionsäure wurde auf 60 °C erwärmt und danach wurden 112 kg (1044,8 Mol), bei 80 °C aufgeschmolzenes p-Toluidin eingesaugt. Der Gasraum über dem Reaktionsgemisch wurde 3 mal mit Stickstoff gespült und der Ansatz am Umlauf gekocht, bis das Kondensationswasser vollständig ausgekreist war. Die Temperatur des Reaktionsgemisches im Kessel wurde auf 100 °C eingestellt und die Kreiselhomogenisierungspumpe angestellt. Es wurden ca. 8,5 m³/h umgepumpt, d. h. der Inhalt des Rührkessels wurde ca. 7 mal pro Stunde umgepumpt. Desweiteren wurde wie im Beispiel 1 verfahren. Obwohl das pro Stunde von der Kreiselhomogenisiermaschine umgepumpte Volumen aufgrund des im Vergleich zu Beispiel 3 3,3-fachen Ansatzes nur ca. 30 % betrug, war die Oxidation bereits nach 13 Stunden beendet. Es wurden 11,8 kg Sauerstoff (Theorie 7,95 kg) verbraucht.
   Die Ausbeute an 2,5-Di-p-toluidino-terephthalsäure-dimethylester betrug über 98 % (HPLC-analytische Bestimmung wie im Beispiel 1).

## Patentansprüche

1. Verfahren zur Herstellung von 2,5-Di-(phenylamino)-terephthalsäure und ihrer Dialkylester der allgemeinen Formel (I), 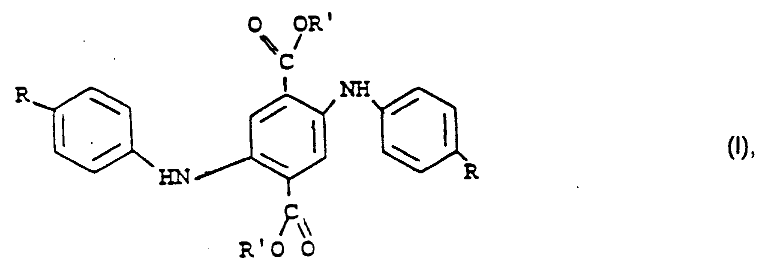 in welcher R ein Wasserstoffatom oder eine Methylgruppe und R' ein Wasserstoffatom oder eine Methyl- oder Ethylgruppe bedeuten, durch (1) Umsetzung von Bersteinsäure-dialkyl(C₁-C₂)-ester nach Art einer Dieckmann-Kondensation mit Natrium-alkoholat in Xylol zum Dinatrium-Salz des 2,5-Dihydroxy-cyclohexadien-carbonsäure-(1,4)-dialkyl(C₁-C₂)esters, (2) Umsetzung des so erhaltenen Kondensationsprodukts nach Zersetzen des Dinatrium-Salzes mit Säure mit einem Phenylamin der allgemeinen Formel (II) 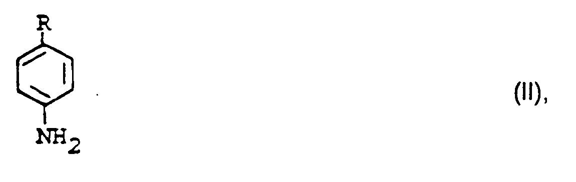 in welcher R die vorstehend genannte Bedeutung hat, in Gegenwart einer organischen Säure in Xylol zum 2,5-Di-(phenylamino)-dihydro(3,6)-terephthalsäuredialkyl(C₁-C₂)-ester, (3) Dehydrierung (Oxidation) des so erhaltenen Cyclohexadien-1,4-derivats mit Sauerstoff zum entsprechenden 2,5-Di-(phenylamino)-terephthalsäure-dialkyl(C₁-C₂)-ester, (4) Verseifung des so erhaltenen Dialkylesters in methanolischer Natronlauge zum entsprechenden 2,5-di-(phenylamino)-terephthalsauren Dinatrium-Salz und (5) Freisetzung der 2,5-Di-(phenylamino)-terephthalsäure aus dem genannten Dinatriumsalz mit Säure, dadurch gekennzeichnet, daß man die Oxidation in Stufe (3) so durchführt, daß man das als Lösung oder Suspension vorliegende Reaktionsgemisch der Stufe (2) in einem Rührkessel mit Stickstoff überlagert, dieses Reaktionsgemisch im Kreislauf umpumpt und den Sauerstoff in ein Teilvolumen des umgepumpten Reaktionsgemisches so eindosiert, daß eine effektive Durchmischung erfolgt.

2. Verfahren nach Anspruch 1, dadurch gekennzeichnet, daß gleichzeitig mit der Eindosierung des Sauerstoffs vorhandene Feststoffteilchen zerkleinert werden.

3. Verfahren nach Anspruch 1 oder 2, dadurch gekennzeichnet, daß die Durchmischung des Reaktionsgemisches mit dem Sauerstoff und/oder die Zerkleinerung der Feststoffteilchen durch Kavitationseffekte bewirkt wird.

4. Verfahren nach einem oder mehreren der Ansprüche 1 bis 3, dadurch gekennzeichnet, daß es sich bei dem Teilvolumen, in das der Sauerstoff eindosiert wird, um den Funktionsraum einer Kreiselhomogenisierungspumpe handelt.

## Claims

1. A process for the preparation of 2,5-di(phenylamino)terephthalic acid and its dialkyl esters of formula (I) 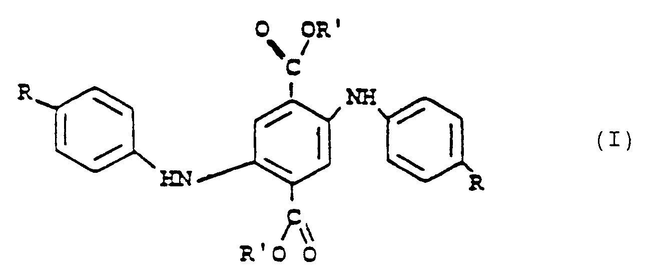 in which R is a hydrogen atom or a methyl group and R' is a hydrogen atom or a methyl or ethyl group, by (1) reacting a (C₁-C₂)-dialkyl succinate with a sodium alcoholate in xylene, in the manner of a Dieckmann condensation, to give the disodium salt of the (C₁-C₂)-dialkyl 2,5-dihydroxycyclohexadiene-1,4-dicarboxylate, (2) reacting the resulting condensation product, after decomposition of the disodium salt with acid, with a phenylamine of formula (II) 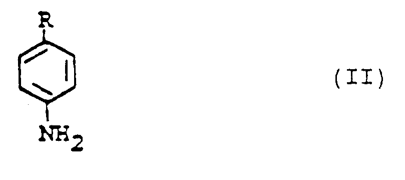 in which R is as defined above, in the presence of an organic acid, in xylene, to give the (C₁-C₂)-dialkyl 2,5-di(phenylamino)-3,6-dihydroterephthalate, (3) dehydrogenating (oxidizing) the resulting 1,4-cyclohexadiene derivative with oxygen to give the corresponding (C₁-C₂)-dialkyl 2,5-di(phenylamino)terephthalate, (4) saponifying the resulting dialkyl ester in methanolic sodium hydroxide solution to give the corresponding disodium 2,5-di(phenylamino)terephthalate, and (5) liberating the 2,5-di(phenylamino)terephthalic acid from said disodium salt with acid, which process comprises carrying out the oxidation in stage (3) in such a way that the reaction mixture of stage (2), in the form of a solution or suspension, is blanketed with nitrogen in a stirred vessel, this reaction mixture is circulated by pumping and the oxygen is metered into a partial volume of the circulated reaction mixture in such a way as to produce effective thorough mixing.

2. The process as claimed in claim 1, wherein solid particles present are comminuted at the same time as the oxygen is metered in.

3. The process as claimed in claim 1 or 2, wherein the thorough mixing of the reaction mixture with the oxygen and/or the comminution of the solid particles are brought about by cavitation effects.

4. The process as claimed in one or more of claims 1 to 3, wherein the partial volume into which the oxygen is metered is the operating space of a centrifugal homogenization pump.

## Revendications

1. Procédé pour la préparation d'acide 2,5-di-(phénylamino)-téréphtalique et de ses esters dialkyliques de formule générale (I) 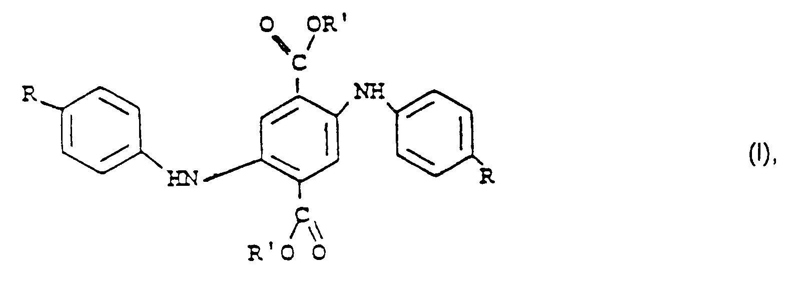 dans laquelle R représente un atome d'hydrogène ou un groupe méthyle et R' un atome d'hydrogène ou un groupe méthyle ou éthyle, (1) par réaction d'un ester dialkylique en C₁-C₂ de l'acide succinique selon une condensation de type de Dieckmann avec un alcoolate de sodium dans du xylène pour obtenir un sel disodique de l'ester (1,4)-dialkylique en C₁-C₂ de l'acide 2,5-dihydroxy-cyclohexadiène-dicarboxylique (2) par réaction du produit de condensation ainsi obtenu après la décomposition du sel disodique avec l'acide, avec une phénylamine de formule générale (II) 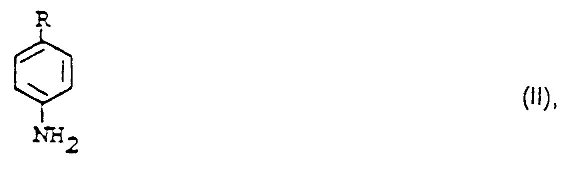 dans laquelle R a la signification donnée précédemment, en présence d'un acide organique dans du xylène pour donner l'ester dialkylique en C₁-C₂ de l'acide 2,5-di-(phénylamino)-dihydro(3,6)-téréphtalique, (3) par déshydrogénation (oxydation) du dérivé du cyclohexadiène-1,4 avec l'oxygène pour obtenir l'ester dialkylique en C₁-C₂ de l'acide 2,5-di-(phénylamino)-téréphtalique correspondant, (4) par saponification de l'ester dialkylique ainsi obtenu dans de la lessive de soude méthanolée pour obtenir le sel disodique de l'acide 2,5-di-(phénylamino)-téréphtalique correspondant et (5) par libération de l'acide 2,5-di-(phénylamino)-téréphtalique à partir du sel disodique cité avec l'acide, caractérisé en ce qu'on effectue l'oxydation dans l'étape (3) de telle façon qu'on recouvre avec l'azote le mélange réactionnel de l'étape (2) présent sous forme de solution ou de suspension dans un réacteur agité, on transfère par pompage ce mélange réactionnel dans le circuit et on ajoute progressivement de l'oxygène dans un volume partiel du mélange réactionnel transféré par pompage de telle façon, qu'il s'en suive un brassage efficace.

2. Procédé selon la revendication 1, caractérisé en ce que simultanément à l'introduction progressive de l'oxygène on fragmente les particules de matière solide présentes.

3. Procédé selon la revendication 1 ou 2, caractérisé en ce qu'on effectue le brassage du mélange réactionnel avec l'oxygène et/ou la fragmentation des particules solides par des effets de cavitation.

4. Procédé selon une ou plusieurs des revendications 1 à 3, caractérisé en ce que dans le cas du volume partiel dans lequel on introduit progressivement l'oxygène, il s'agit de l'espace opérationnel d'une pompe d'homogénéisation gyroscopique.
